(19) Europäisches Patentamt
European Patent Office
Office européen des brevets

(11) EP 0 781 556 A1

(12) EUROPÄISCHE PATENTANMELDUNG

(43) Veröffentlichungstag:
**02.07.1997 Patentblatt 1997/27**

(51) Int. Cl.[6]: **A61K 31/435**, A61K 31/47

(21) Anmeldenummer: **96119881.9**

(22) Anmeldetag: **20.12.1991**

(84) Benannte Vertragsstaaten:
**AT BE CH DE DK ES FR GB GR IT LI LU NL SE**

(30) Priorität: **22.12.1990 DE 4041482**

(62) Dokumentnummer(n) der früheren Anmeldung(en) nach Art. 76 EPÜ:
**92901178.1 / 0 563 128**

(71) Anmelder:
- **BOEHRINGER INGELHEIM KG**
  **55216 Ingelheim (DE)**
  Benannte Vertragsstaaten:
  **BE CH DE DK ES FR GR IT LI LU NL SE AT**
- **BOEHRINGER INGELHEIM INTERNATIONAL GmbH**
  **D-55216 Ingelheim am Rhein (DE)**
  Benannte Vertragsstaaten:
  **GB**

(72) Erfinder:
- **Loesel, Walter**
  **55435 Gau-Algesheim (DE)**
- **Roos, Otto**
  **55270 Schwabenheim (DE)**
- **Arndts, Dietrich**
  **55437 Appenheim (DE)**
- **Kuhn, Franz Josef**
  **55218 Ingelheim (DE)**
- **Streller, Ilse**
  **55442 Stromberg (DE)**

Bemerkungen:
Diese Anmeldung ist am 11 - 12 - 1996 als Teilanmeldung zu der unter INID-Kode 62 erwähnten Anmeldung eingereicht worden.

# (54) Verwendung von kondensierte Dihydropyridine zur Behandlung von chronisch inflammatorische Prozesse

(57) Die Erfindung betrifft die Verwendung von carbocyclisch und heterocyclisch anellierten Dihydropyridinen der Formel

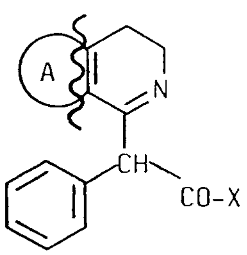

I

sowie deren tautomere Formen der Formel II

EP 0 781 556 A1

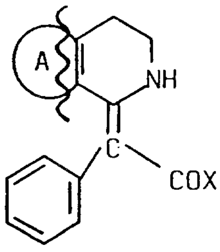

oder

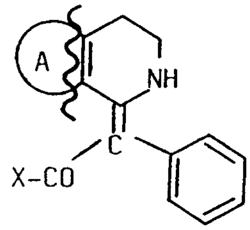

II'

II''

worin A für die anellierten Ringsysteme

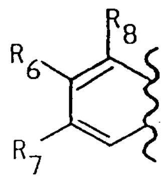

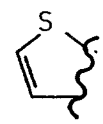

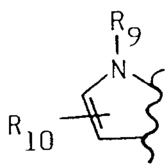

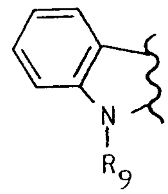

steht und X für $OR_1$, $NHR_2$ oder $NR_3R_4$, für die Herstellung von Mitteln für die Behandlung chronisch inflammatorischer Prozesse.

## Beschreibung

Aus der EP-A 37 934 sind Dihydroisochinoline der allgemeinen Formel Ia bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden. Diese Verwendungsmöglichkeiten beruhen auf der vaskulären Wirksamkeit der Verbindungen. In der EP-A 251 194 ist beschrieben worden, daß carbocyclisch und heterocyclisch annelierte Dihydropyridine cardioprotektive Wirkung besitzen und einen völlig neuen Typ Ca-antagonistischer Verbindungen verkörpern.

Gegenstand der vorliegenden Erfindung ist die Verwendung der aus der genannten EP-A 251 194 bekannten Verbindungen und strukturell ähnlicher Verbindungen für die Behandlung chronisch inflammatorischer Prozesse (z.B. Bronchialasthma, Arthritis).

Die Erfindung betrifft somit die Verwendung von carbocyclisch und heterocyclisch anellierten Dihydropyridinen der Formel

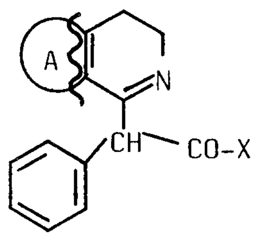

I

worunter auch deren tautomere Formen der Formel II erfaßt werden, wobei diese Bezeichnung zusammenfassend für die cis- und trans-Formen II' und II" steht:

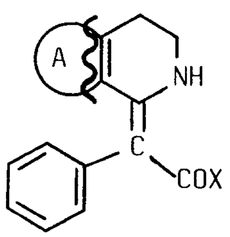

oder

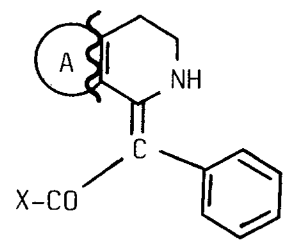

II' II''

II

in der X für $OR_1$; $NHR_2$; $NR_3R_4$
und

$R_1$ für Wasserstoff, $C_1$-$C_4$-Alkyl; $C_1$-$C_4$-Alkoxyalkyl

$R_2$ für Wasserstoff; $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c), die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann),

b) Phenyl; gewünschtenfalls ein- oder mehrfach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe -O-$(CH_2)_n$-O (mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol

(wobei die zuvor genannen Heterocyclen ein- oder mehrfach durch $C_1$-$C_4$-Alkyl substituiert sein können,) $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

oder $R_2$ Phenyl ist, das wie oben unter (b) angegeben substituiert sein kann;

$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1) substituiert sein kann

und

$R_5$ für ein Phenylradikal, welches gewünschtenfalls wie vorstehend unter b) substituiert ist, steht;

A steht für die anellierten Ringsysteme

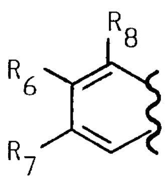

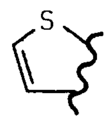

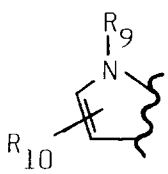

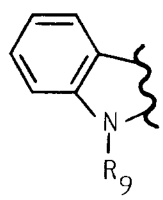

in denen

$R_8$ für Wasserstoff; $C_1$-$C_4$-Alkyl; oder $C_1$-$C_4$-Alkoxy

$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für $-O-(CH_2)_n-O-$ (mit n = 1 oder 2)

$R_9$ für Wasserstoff; $C_1$-$C_4$-Alkyl

$R_{10}$ für Wasserstoff; oder 2-Phenyl-2-ethoxycarbonylacetyl

steht sowie deren Salze mit physiologisch verträglichen Säuren, Basen oder Komplexbildnern für die Behandlung chronisch inflammatorischer Prozesse.

Unter die beschriebene allgemeine Formel I fallen auch die neuen 3,4-Dihydroisochinolinderivate der allgemeinen Formel Ie

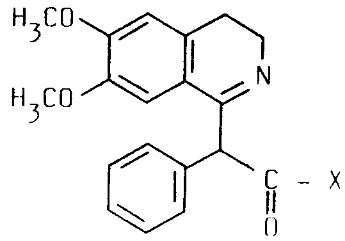

(Ie)

worin X

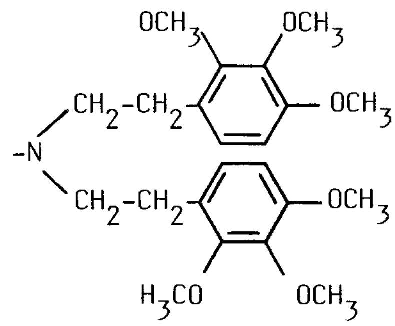

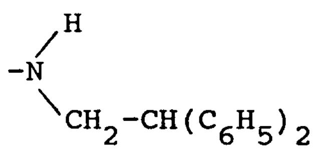

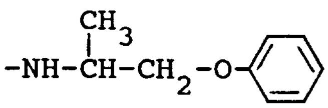

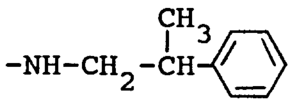

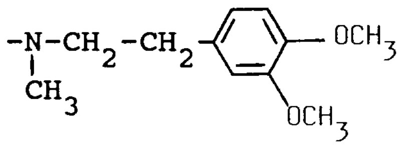

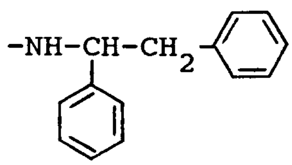

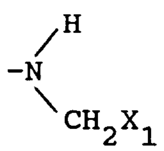

oder

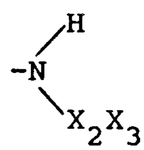

ist,
worin

$X_1$ durch Trifluormethyl oder Ethoxy mono- oder di-substituiertes Phenyl, durch Methoxy und Fluor substituiertes Phenyl oder 2-Methoxyphenyl ist,

$X_2$ $-CH_2-CH_2-$ oder $-CH_2-CH(CH_3)-$ ist,
und

$X_3$ 2,3,4-Trimethoxyphenyl, 2,3-Dimethoxyphenyl, 2,6-Dimethoxyphenyl, 3,6-Dimethoxyphenyl, Thienyl, durch Trifluormethyl oder Ethoxy mono- oder di-substituiertes Phenyl, oder durch Methoxy und Fluor substituiertes Phenyl ist,

oder deren pharmazeutisch annehmbare Salze. Diese Verbindungen sind in der Stammanmeldung dieser Teilanmeldung (Nr. 92901178.1) beschrieben.
Bevorzugt sind Verbindungen (Ie),
worin X

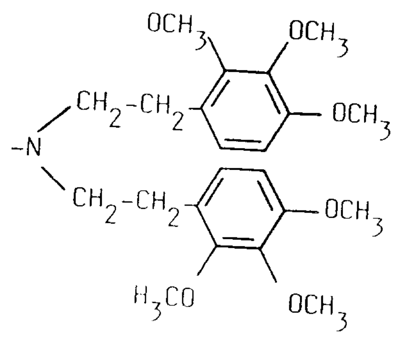

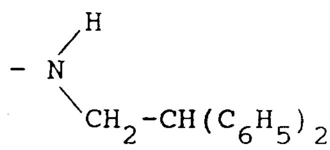

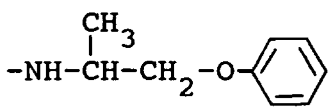

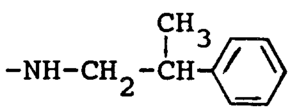

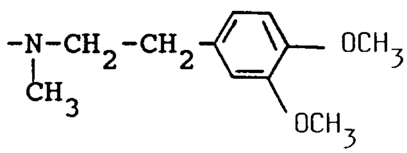

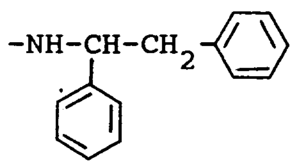

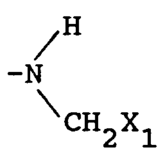

oder

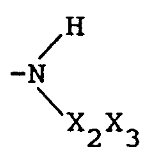

ist,
worin

$X_1$ 2-Methoxyphenyl ist, das zusätzlich durch Fluoro subsituiert sein kann,

$X_2$ $-CH_2-CH_2$ - ist
und

$X_3$ 2,3,4-Trimethoxyphenyl, 2,3-Dimethoxyphenyl, 2,6-Dimethoxyphenyl, 3,6-Dimethoxyphenyl, 2 oder 3-Thienyl, durch Trifluormethyl oder Ethoxy substituiertes Phenyl, oder durch Methoxy und Fluor substituiertes Phenyl ist.

Diese neuen Verbindungen sind auch für die oben beschriebenen Verwendung geeignet.
Bevorzugt ist die Verwendung von carbocyclisch und heterocyclisch anellierten Dihydropyridinen der Formel

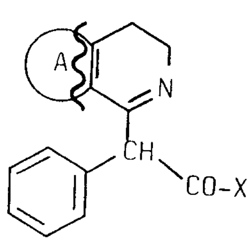

I

sowie deren tautomere Formen der Formel

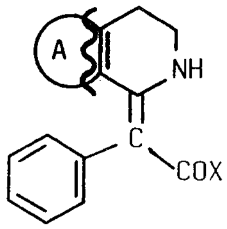

oder

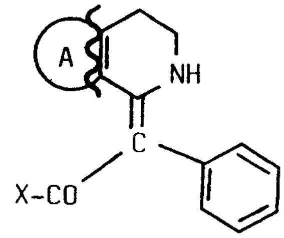

II'   II''

II

in der X für $OR_1$ $NHR_2$; $NR_3R_4$

$R_1$ für Methyl oder Ethyl

$R_2$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

d) Cyano, Hydroxy, Methoxy, Dimethylamino

e) Phenyl, 3,4-Methylendioxyphenyl, durch eine, zwei odere 3 Methoxygruppen substituiertes Phenyl, 3-Hydroxy-4-methoxyphenyl,

f) Morpholino, Pyridin-2-yl, Indol-3-yl, Furan-2-yl, Thiophen-2-yl, Pyridin-3-yl, Pyridin-4-yl

$R_3$ und $R_4$ unabhängig voneinander für Methyl; Ethyl; 3-Cyanopropyl; Benzyl; oder 3,4,5-Trimethoxyphenethyl stehen oder

$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und

A für die anellierten Ringsysteme

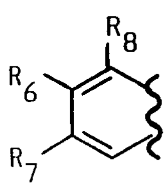

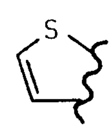

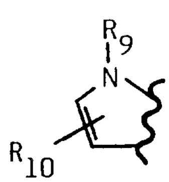

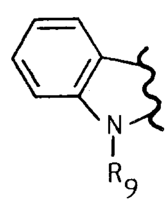

in denen

$R_8$ für Wasserstoff; oder Methoxy

$R_6$ für Methoxy; Hydroxy; Wasserstoff; Amino; oder Methansulfonylamino

$R_7$ für Wasserstoff; Methoxy; oder Hydroxy

$R_9$ für Methyl

$R_{10}$ für 2-Phenyl-2-ethoxycarbonylacetyl; oder Wasserstoff

steht, sowie deren Salze mit physiologisch verträglichen Säuren.

Die Erfindung betrifft somit Dihydroisochinoline der Formel

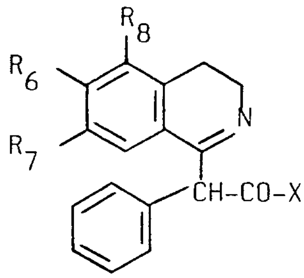

Ia

Dihydro-thieno[3,2-c]pyridine der Formel

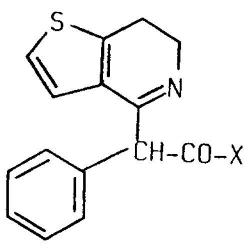

Ib

Dihydro-pyrollo[3,2-c]pyridine der Formel

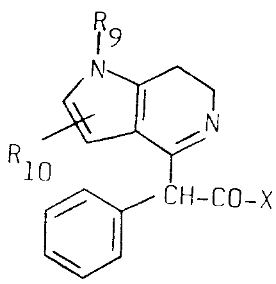

und Dihydro-pyrido[3,4-b]indole der Formel

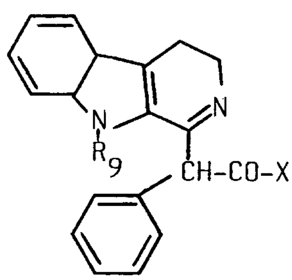

Id

sowie deren tautomere Formen IIa', IIb', IIc' und IId' und die dazu E/Z-isomeren Formen IIa'', IIb'', IIc'' und IId''.

Die Verbindungen der Formel Ia - Id sind chiral. Die Erfindung umfaßt auch die beiden R- und S-enantiomeren Formen der Verbindungen der Formel I bei denen die Bedeutung der Reste X, $R_6$, $R_7$, $R_8$, $R_9$ und $R_{10}$ wie zuvor definiert ist.

Die in den nachstehenden Tabellen 1 bis 12 aufgeführten Substanzen liegen jeweils bevorzugt in der tautomeren Form I oder II vor.

Die bevorzugte tautomere Form ist in der Spalte Struktur mit I bzw. II gekennzeichnet.

Namentlich zu nennen sind folgende Verbindungen der Formel I in der tautomeren Form I bzw. II.

In Tabelle 12 sind neue Verbindungen der allgemeinen Formel Ie zusammengefaßt.

Tabelle 1

Strukturtyp:

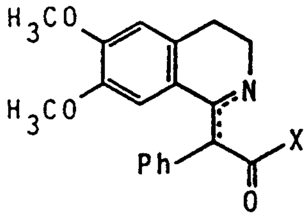

| Nr. | X | Struktur | Salzform |
|---|---|---|---|
| 1. | $OCH_3$ | II | - |
| 2. | $OC_2H_5$ | II | - |
| 3. | $NHCH_3$ | I | Cl |
| 4. | $NHC_2H_5$ | I | - |
| 5. | $NH-(CH_2)_2-CH_3$ | I | Cl |
| 6. | $NH-(CH_2)_3-CH_3$ | I | Cl |
| 7. | $NH-(CH_2)_4-CH_3$ | I | Cl |
| 8. | $NH-CH(CH_3)_2$ | I | Cl |
| 9. | $NH-CH_2-CH(CH_3)_2$ | I | Cl |
| 10. | $NH-(CH_2)_2-CH(CH_3)_2$ | I | Cl |
| 11. | $NH-C(CH_3)_3$ | I | Cl |
| 12. | $NH-CH(CH_3)-C_2H_5$ | I | Cl |
| 13. | $NH-CH_2-CH=CH_2$ | I | Cl |
| 14. | $NH-CH_2-C\equiv CH$ | I | Cl |
| 15. | $NH-(CH_2)_2-OH$ | II | Cl |
| 16. | $NH-CH_2-CH(OH)-CH_3$ | I | Cl |
| 17. | $NH-(CH_2)_2-OCH_3$ | II | Cl |
| 18. | $NH-(CH_2)_3-OCH_3$ | II | Cl |
| 19. | $NH-(CH_2)_2-N(CH_3)_2$ | I | $Cl_2$ |
| 20. | $NH-(CH_2)_3-N(CH_3)_2$ | II | $Cl_2$ |
| 21. | $NH-(CH_2)_2-$ 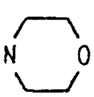 | II | - |

| Nr. | X | Struktur | Salzform |
|---|---|---|---|
| 22. | $NH-(CH_2)_2-$ 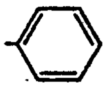 | I | Cl |
| 23. | $NH-(CH_2)_2-$ 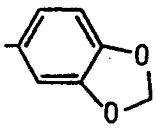 | I | - |
| 24. | $NH-(CH_2)_2-$ 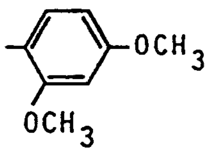 | I | - |
| 25. | $NH-(CH_2)_2-$ 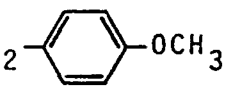 | I | - |
| 26. | $NH-(CH_2)_2-$ 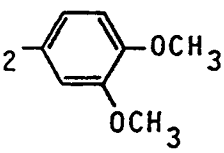 | II | - |
| 27. | $NH-(CH_2)_2-$ 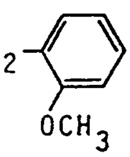 | II | Cl |
| 28. | $NH-(CH_2)_2-$ 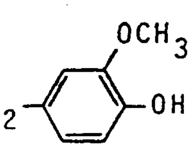 | I | - |
| 29. | $NH-(CH_2)_2-$ 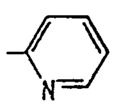 | I | - |

| Nr. | X | Struktur | Salzform |
|---|---|---|---|
| 30. | 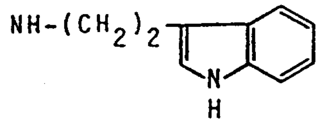 | II | - |
| 31. | 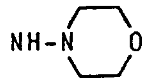 | I | - |
| 32. | 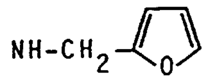 | II | - |
| 33. | 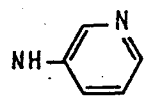 | I | Cl |
| 34. | 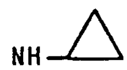 | I | Cl |
| 35. | 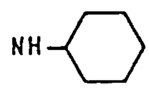 | II | - |
| 36. | $N(CH_3)_2$ | I | Cl |
| 37. | $N(C_2H_5)_2$ | I | Cl |
| 38. | 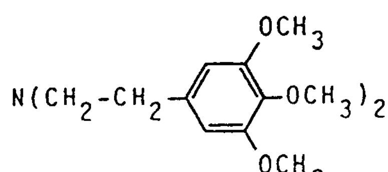 | I | Cl |
| 38a. | 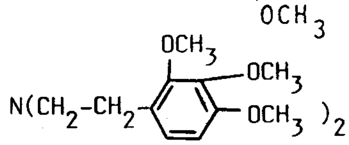 | I | Cl |
| 39. | 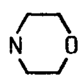 | I | Cl |

| Nr. | X | Struktur | Salzform |
|---|---|---|---|
| 40 | 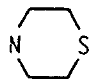 | I | - |
| 41. | 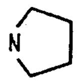 | I<br>II | - |
| 42. | 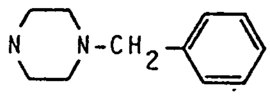 | II | $Cl_2$ |
| 43 | 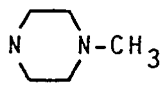 | I | - |
| 44 | 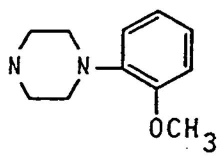 | I | Cl |

Tabelle 2

Strukturtyp:

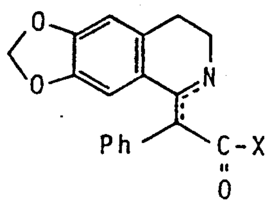

| Nr. | X | Struktur | Salzform |
|---|---|---|---|
| 45. | $OC_2H_5$ | II | - |
| 46. | $NH-(CH_2)_2-$ 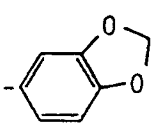 | II | Cl |
| 47. | $NH-(CH_2)_2$ 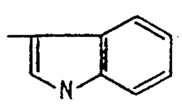 | II | - |

| Nr. | X | Struktur | Salzform |
|---|---|---|---|
| 48. | NH-$(CH_2)_2$- 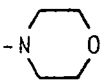 | II | - |
| 49. | 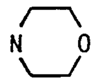 | II | - |
| 50. | 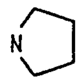 | I | - |

Tabelle 3

Strukturtyp:

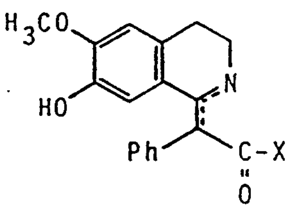

| Nr. | X | Struktur | Salzform |
|---|---|---|---|
| 51. | $OC_2H_5$ | I<br>II | - |
| 52. | NH-$(CH_2)_3$-$CH_3$ | I | - |
| 53. | NH-$(CH_2)_4$-$CH_3$ | I | - |
| 54. | NH-$CH(CH_3)_2$ | I | Cl |
| 55. | NH-$CH_2$-$CH(CH_3)_2$ | I | - |
| 56. | NH-$CH_2$-CH=$CH_2$ | I | - |
| 57. | NH-$(CH_2)_2$- 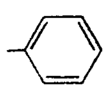 | I | Cl |
| 58. | NH-$(CH_2)_2$- 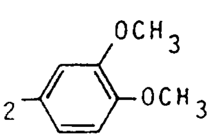 | I | Cl |

| Nr. | X | Struktur | Salzform |
|---|---|---|---|
| 59. | 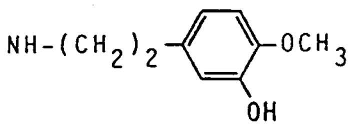 | I | |
| 60. | 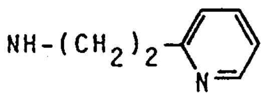 | I | |
| 61. | 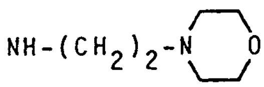 | I | – |
| 62. | 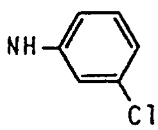 | I | – |
| 63. | 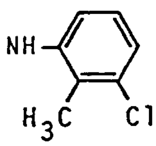 | I | Cl |
| 64. | 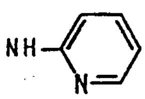 | I | – |
| 65. | 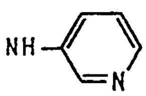 | I | Cl |
| 66. | 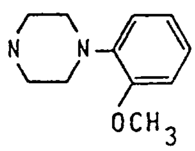 | I | Cl |
| 67. | 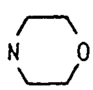 | I | Cl |
| 68. | 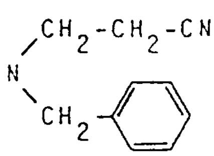 | I | Cl |

Tabelle 4

Strukturtyp:

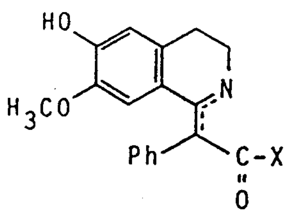

| Nr. | X | Struktur | Salzform |
|---|---|---|---|
| 69. | $N(C_2H_5)_2$ | I | |

Tabelle 5

Strukturtyp:

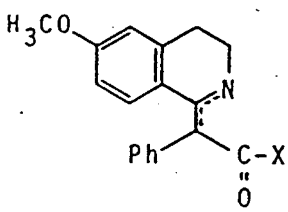

| Nr. | X | Struktur | Salzform |
|---|---|---|---|
| 70. | $NHCH_3$ | I | |
| 71. | $NHC_2H_5$ | I | |
| 72. | $NH-CH_2-$ 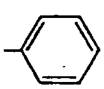 | I | |
| 73. | $NH(CH_2)_2-$ 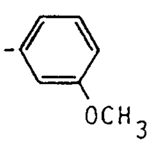 | I | |
| 74. | $N(CH_3)C_2H_5$ | I | |

Tabelle 6

Strukturtyp:

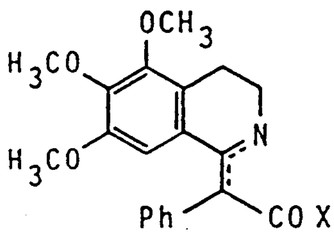

| Nr. | X | Struktur |
| --- | --- | --- |
| 75. | 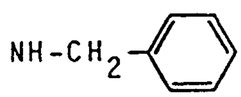 | I |
| 76. | 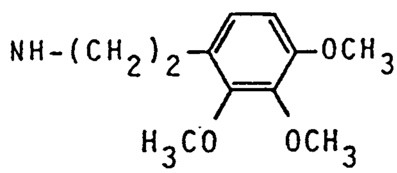 | I |
| 77. | $N(CH_3)C_2H_5$ | I |
| 78. | 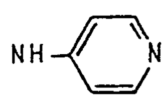 | II |
| 79. | 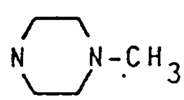 | I |

Tabelle 7

Strukturtyp:

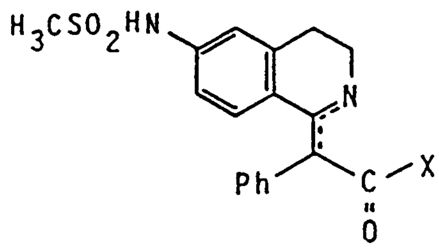

| Nr. | X | Struktur | Salzform |
|---|---|---|---|
| 80 | $OC_2H_5$ | II | - |

Tabelle 8

Strukturtyp:

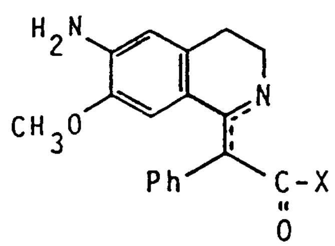

| Nr. | X | Struktur | Salzform |
|---|---|---|---|
| 81. | $OC_2H_5$ | II | Cl |

Tabelle 9

Strukturtyp:

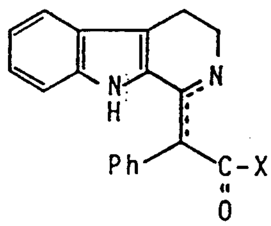

| Nr. | X | Struktur | Salzform |
|---|---|---|---|
| 82. | $OC_2H_5$ | I<br>II | - |
| 83. | $NH-(CH_2)_2-$ 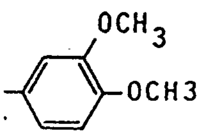 | II | - |
| 84. | $NH-(CH_2)_2-$ 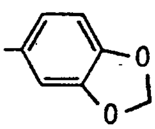 | II | - |
| 85. | $NH-(CH_2)_2-$ 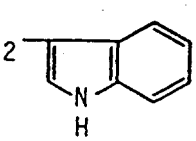 | I<br>II | - |
| 86. | 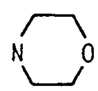 | I | - |
| 87. | $NH-CH_2-CH(CH_3)_2$ | II | - |
| 88. | $NH-(CH_2)_3-N(CH_3)_2$ | II | - |
| 89. | $NH-(CH_2)_2-$ 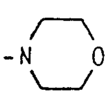 | II | - |

Tabelle 10

Strukturtyp:

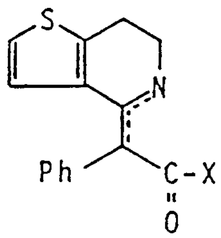

| Nr. | X | Struktur | Salzform |
|---|---|---|---|
| 90. | $OC_2H_5$ | | |
| 91. | NH-$(CH_2)_2$-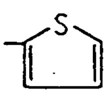 | II | - |

Tabelle 11

Strukturtyp:

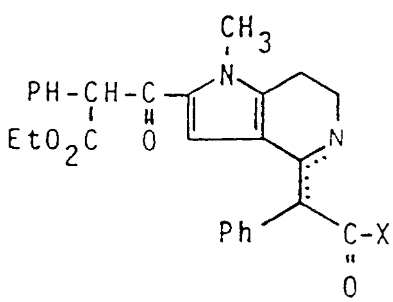

| Nr. | X | Struktur | Salzform |
|---|---|---|---|
| 92. | $OC_2H_5$ | II | - |

Tabelle 12

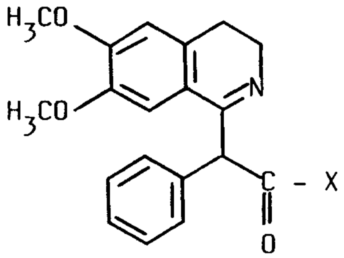

Strukturtyp I

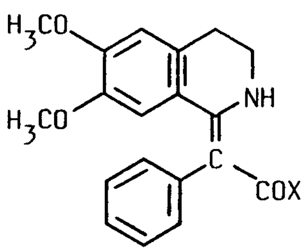

oder

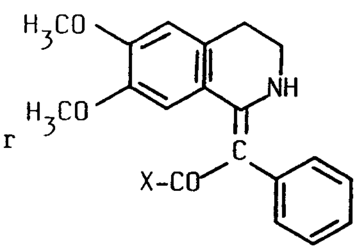

II'

II''

Strukturtyp II

| Verbindung | X | Strukturtyp | Fp[°C] |
|---|---|---|---|
| A | 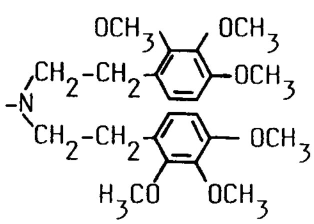 .HCl | I | 56-64 |
| B | 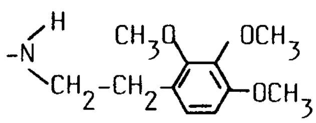 .HCl | I | 176-184 |

| Verbindung (Salzform) | | X | Strukturtyp | Fp[°C] |
|---|---|---|---|---|
| C | | 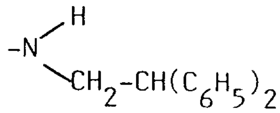 | I | 166-168 |
| D | | 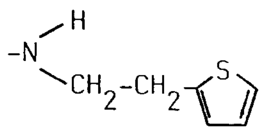 | II | 102-104 |
| E | (Cl) | 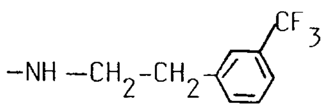 | I | 187 |
| F | (-) | 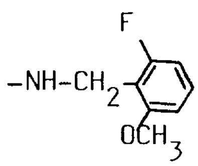 | I | 94-96 |
| G | (Cl) | 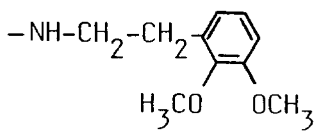 | II | 139-142 |
| H | (-) | 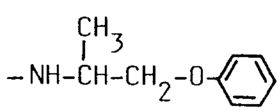 | II | 133-135 |
| J | (-) | 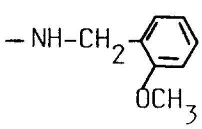 | II | 143-145 |
| K | (-) | 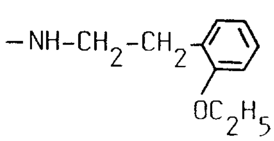 | II | 96-98 |

| Verbindung (Salzform) | | X | Strukturtyp | Fp[°C] |
|---|---|---|---|---|
| L | (-) | 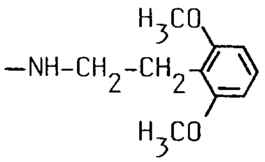 | II | 118-120 |
| M | (-) | 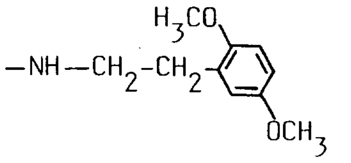 | II | 112-114 |
| N | (Cl) | 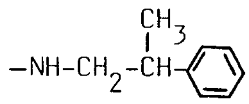 | I | 95-99 |
| O | (-) | 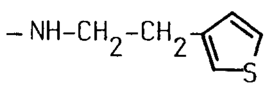 | I | 114-116 |
| P | (-) | 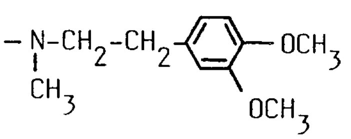 | I | 66-73 |
| Q | (Cl) | 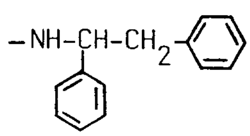 | II | 205-209 |

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind $C_1$-$C_6$-Alkyl- und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl-, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Die vorstehende Definition gilt somit auch wenn das Alkylradikal seinerseits substituiert und/oder selbst Bestandteil einer Alkoxyalkyl-, Alkoxycarbonyl-, Alkoxy-, Alkylthio-, Alkylsulfonyl-, Monoalkylamino-, Alkylmethyl-, Alkylthiomethyl-, Dialkylaminogruppe ist oder das Alkylradikal als Substituent an ein aromatisches heterocyclisches oder carboxyclisches System gebunden ist.

Halogene sind Fluor, Chlor, Brom und Jod, vorzugsweise Fluor, Chlor und Brom und in zweiter Linie Jod.

$C_3$-$C_6$-Cycloalkyl sind Cyclopropan, Cyclobutan, Cyclopentan und Cyclohexan.

$C_5$-$C_6$-Cycloalkene sind z.B. Cyclopenten, Cyclohexen und Cyclohexadien.

Das $C_2$- und $C_3$-Acylradikal steht für das Acetyl und das Propionylradikal.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl

Als ungesättigte Heterocyclen sind unter anderem zu nennen:
Furan, Pyran, Pyrrol, Pyrazol, Imidazol, Pyridin, Pyrazin, Pyrimidin, Pyridazin, Thiophen, Thiazol, Oxazol, 1,2,4-Triazol, 1,2,3-Triazol, 1,2,4-Triazin, 1,3,5-Triazin, Indol.

Als 5- oder 6-gliedrige ganz oder teilweise gesättigte monocyclische Heterocyclen sind unter anderem zu nennen: Imidazolidin, Pyrazolidin, Pyrrolidin, Piperidin, Piperazin, Morpholin, Thiomorpholin, Tetrahydrofuran, Tetrahydrothiophen, 1,4-Dioxin, Imidazolin, Pyrazolin, Pyrrolin, etc.

Die Verbindungen der Formel I bzw. II sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Die meisten Verbindungen der allgemeinen Formel I und deren Herstellungsmethoden sind aus den eingangs genannten EP-A 37 934 und EP-A 251 194 bekannt. In diesen Veröffentlichungen sind die Verbindung der Formel Ie nicht genannt.

Wie bereits eingangs erwähnt ist der Gegenstand der vorliegenden Erfindung die Verwendung der aus der genannten EP-A 251 194 bekannten Verbindungen und insbesondere der neuen Verbindung des Beispiels 38A als hirnprotektive Mittel. Die Verbindungen sind zur Behandlung von degenerativen und nekrotischen Erkrankungen des Gehirns vorteilhaft. Ebenso ist die vorbeugende Behandlung von durch solche Krankheiten gefährdeten Patienten möglich. Wie durch die weiter unten beschriebenen Versuche gezeigt wird, beruht die Wirkung der Verbindungen nicht auf einer Verbesserung der Gewebsdruchblutung. Die Verbindungen sind somit für eine neuartige Behandlung von Epilepsie und der Alzheimer-Krankheit geeignet, und insbesondere bei der Behandlung von Patienten, die einen Schlaganfall erlitten haben oder gefährdet sind, einen Schlaganfall zu erleiden.

Ferner sind die Verbindungen, wie eingangs erwähnt, für die Behandlung chronisch inflammatorischer Prozesse und für die Hemmung der Blutgerinnung geeignet.

Die folgenden Untersuchungsergebnisse zeigen die überraschende Wirkung der Verbindungen. Die Untersuchungen wurden insbesondere mit Verbindung A (Beispiel 38A)

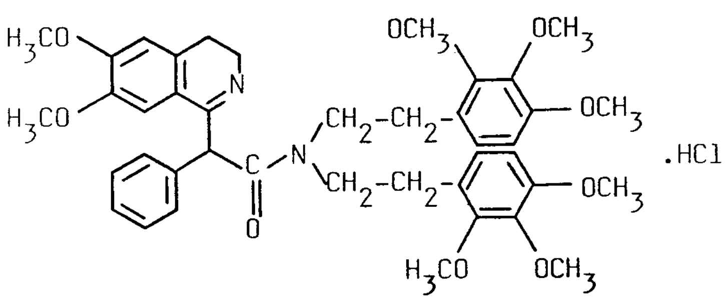

als Vertreterin der Verbindungen der allgemeinen Formel I ausgeführt.

Ischämietoleranz am Gerbil:
(z.B. Suzuki, R. et al., Acta Neuropath (Berl.) 1983, 60:207-216, 217-222; Yoshidomi, M. et al., J. Neurochem. 1989, 53:1589-1594)
Ischämie durch Occlusion der Arteria carotis für 10 Minuten unter Etheranaesthesie. Die Verbindung A wurde mit 1 und 10 mg/kg s.c. 4 x innerhalb von 24 Stunden, beginnend 2 Stunden nach Wiederöffnung der Arterien, appliziert.

72 Stunden nach Gefäßocclusion wurden die Tiere getötet und die Gehirne zur histologischen Untersuchung präpariert. Als Nachweis eines ischämischen Schadens beziehungsweise dessen Reduzierung wurde im Bereich der $CA_1$-Region des Hippocampus in einem definierten Ausschnitt des histologischen Präparates die Schädigung der Zellen beurteilt. Die Untersuchung wurde an Gruppen von je 5 Tieren ausgeführt.

In der Gruppe, in der den Tieren keine zu untersuchende Verbindung verabreicht wurde, zeigten alle Tiere eine deutliche Schädigung der untersuchten $CA_1$-Region. Im Gegensatz dazu zeigte sich bei Verabreichung der Verbindung A ein deutlicher dosisabhängiger Schutz gegen den Hypoxieschaden. Die Verabreichung von 1 mg/kg der Verbindung A zeigen nur 2 von 5 Tieren Schädigungen der $CA_1$-Region. Bei Verabreichung von 10 mg/kg der Verbindung A zeigt keines der 5 Tiere eine Schädigung.

Aus diesem Ergebnis kann geschlossen werden, daß die Verbindungen der allgemeinen Formel I zur kurativen Behandlung von neurologischen Schäden, z.B. verursacht durch Schlaganfall, verwendet werden können.

Das Ergebnis dieser Untersuchung zeigt auch, daß die Verbindungen die Blut-Hirn-Schranke überwinden, was ein

wesentliches Merkmal der Erfindung ist.

Die folgenden Untersuchungen an isolierten Zellkulturen zeigen die Wirkung der untersuchten Verbindungen. Ferner kann man aus diesen Untersuchungsergebnissen ebenfalls (wie aus den Untersuchungsergebnissen am Gerbil) schließen, daß die Verbindungen der allgemeinen Formel I für die in dieser Patentanmeldung genannten Behandlungen verwendet werden können.

In isolierten Zellkulturen (z.B. neutrophile Granulozyten, HL 60-Zellen, Thrombozyten, u.a.) konnte Verbindung A den durch unterschiedliche Agonisten (z.B. PAF, Leukotriene, Endothelin, FMLP oder Isoprenalin) provozierten "Calciumoverload" und Zelltod dosisabhängig inhibieren ($IC_{50}$-Werte im Bereich von 1 µM).

Gemessen wurden die halbmaximalen Hemmkonzentrationen der Testsubstanzen, die den FMLP (1 nM)-induzierten Calciumtransienten an $FLUO_3$-beladenen HL60-Zellen inhibieren.

| Verbindung Nr. | halbmaximale Hemmkonzentration (Durchschnittswert $10^6$Zellen in Suspension) |
|---|---|
| 43 | $5.10^{-5}$M |
| 47 | $4,8.10^{-5}$M |
| 38 (Verbindung A) | $5,4.10^{-6}$M |

Diese oben erwähnten Untersuchungsmethoden sind in W.K. Pollock, T.J. Rink, R.F. Irvine, Biochem. J. 235:869-877 (1986) beziehungsweise J.E. Merritt, R. Jacob, T.J. Hallam, J. of Biol. Chem. 264:1522-1527 (1989) beschrieben.

An einzelnen HL-60 Zellen wurde mittels patch clamp Technik elektrophysiologisch der transmembranäre Einstrom durch "Unselective Cation Channels" nach ATP Stimulation gemessen. Dieser Einstrom wird durch Verbindung A inhibiert ($IC_{50}$-Wert: 7 nM).

Diese Befunde belegen den direkten Angriffspunkt an isolierten Zellen. Im lebenden Organismus wandern speziell die in weiteren Untersuchungen verwendeten Granulozyten bzw. Leukozyten nach einer Schädigung von Gehirnzellen (z.B. durch Schlaganfall) in das geschädigte Areal ein, wo sie durch Calcium vermittelte Prozesse aktiviert werden, zerfallen und dabei gewebeschädigende, darunter auch chemotaktische Mediatoren freisetzen (z.B. PAF, Leukotriene, Prostaglandine, FMLP u.a.). Durch Chemotaxis angelockte zusätzliche Leukozyten vergrößern das geschädigte Areal. Die oben beschriebenen Untersuchungsergebnisse zeigen, daß dieser Circulus vitiosus durch Verabreichung der oben beschriebenen aktiven Substanzen blockiert werden kann. Dadurch bleibt der neurologische Schaden begrenzt.

Es konnte gezeigt werden, daß klassische Calciumantagonisten (z.B. Verapamil, Nifedipine, Diltiazem) die Leukozytenaktivierung nicht inhibieren.

Weitere Untersuchungen mit Verbindung A unterstützen die obige Feststellung:

An isolierten corticalen und hippocampalen Neuronenzellen aus fetalen Rattenhirnen (Präparation nach H.W. Müller und W. Seifert, Proc. Natl. Acad. Sci. USA 81: 1248-1252, 1984; J. Neurosci. Res. 8: 195-204, 1982; sowie Müller and Seifert in "Methods for Serum Free Culture of Neuronal and Lymphoid Cells," p. 67-77, A.R. Liss Inc., 150 Fifth Ave., New York, N.Y. 10011, 1984) wurde der direkte neuronale Angriff der Verbindung A nachgewiesen. In $FURA_2$-beladenen Einzelzellen wurden die Konzentrations-Zeit-Kurven (Calciumtransient) des cytoplasmatischen $Ca^{2+}$ registriert (Methode: modifiziert nach J.A. Connor, Proc. Natl. Acad. Sci. 83: 6179-6183, 1986). Sowohl nach mechanischer Läsion als auch nach Gabe exzitatorischer Aminosäuren (E.A.A., Glutamat, Kainat, Quisqualat und NMDA) wurde ein starker Anstieg der cytoplasmatischen Calciumkonzentrationen provoziert, der in allen Fällen dosisabhängig ($IC_{50}$ bei 3 µM) durch Verbindung A inhibierbar war.

Der Wirkungsmechanismus dieser Hemmung wurde sowohl an neuronalen Zellkulturen als auch an menschlichen neutrophilen Granulozyten und HL 60-Zellen und Thrombozyten untersucht. Es konnte gezeigt werden, daß Verbindung A den transmembranären Calciumeinstrom in die Zellen hemmt, der durch Rezeptoragonisten (z.B. EAA, FMLP, Leukotriene, PAF, Endothelin u.a.) stimuliert wird. Dieser von T.J. Hallam and T.J. Rink (Tips 10: 8-10, 1989) als "receptor mediated $Ca^{2+}$ entry (RMCE)" bezeichnete Influx ist durch klassische Calciumantagonisten nicht inhibierbar. Klassische Calciumantagonisten können die Leukozyten und Thrombozyten-Aktivierung nicht verhindern, da diese Zellen keine spannungsabhängigen $Ca^{2+}$-Kanäle haben. Die Blockade des transmembranären Calciumeinstroms konnte elektrophysiologisch (Voltage clamp-Technik) an HL6O-Zellen und Neuronenzellen bestätigt werden.

Die Versuchsergebnisse werden in der folgenden Tabelle zusammengefaßt. Die Verbindungen sind in den Tabellen 1-12 angegeben.

%H = % Hemmung des transmembranären $Ca^{2+}$-Einstroms (Neuronenzellen)

| Verbindung Nr. | % H |
|---|---|
| 2 | 83.78 |
| 6 | 72.79 F |
| 7 | 79.94 F |
| 10 | 67.83 F |
| 16 | 74.58 F |
| 22 | 87.95 F |
| 23 | 72.67 |
| 24 | 91.97 |
| 25 | 82.93 |
| 26 | 46.05 |
| 27 | 93.80 |
| 30 | 78.62 |
| 32 | 61.53 |
| 37 | 65.34 F |
| 42 | 58.78 |
| 45 | 62.72 |
| 46 | 82.72 |
| 47 | 52.29 |
| 57 | 54.19 |
| 83 | 76.84 |
| 87 | 61.91 |
| 89 | 53.29 |
| 91 | 66.76 |
| 92 | 42.12 |

| Verbindung Nr. | % H |
|---|---|
| A | 88.08 |
| B | 64.15 |
| C | 86.93 |
| D | 69.41 |
| E | 93.19 |
| F | 76.64 |
| G | 67.72 |
| H | 79.69 |
| J | 83.81 |
| K | 98.89 |
| L | 99.38 |
| M | 73.21 |
| N | 98.27 |
| O | 90.34 |
| P | 74.58 |
| Q | 81.86 |

Aus diesen Versuchsergebnissen kann geschlossen werden, daß für die Herstellung hirnprotektiver Mittel die Verwendung von Verbindungen der allgemeinen Formel I vorteilhaft ist, worin A

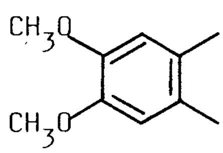

oder

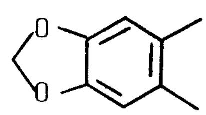

ist,

X $OC_2H_5$, oder
$NHR_1$, worin $R_1$ Alkyl mit 4 oder 5 Kohlenstoffatomen,

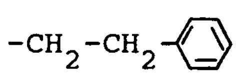

(worin die Phenylgruppe durch eine, zwei oder drei Methoxygruppen, $-O-CH_2-O-$, $CF_3$, F, $OC_2H_5$ oder

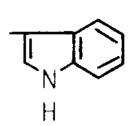

substituiert sein kann) oder $R_1$ $-CH_2-CH(C_6H_5)_2$ ist,

oder X $NR_3R_4$ ist, insbesondere worin
X $N(C_2H_5)_2$ oder

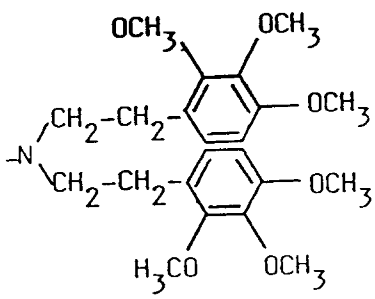

ist.

Die Hemmung der Blutgerinnung bzw. der Blutplättchenaggregation gemäß der Erfindung kann durch Standarduntersuchungen gezeigt werden: An QUIN 2 beladenen menschlichen Blutplättchen, die mit ADP, Vasopressin, PGF2α, Thrombin oder Serotonin stimuliert worden sind, kann gezeigt werden, daß der intrazelluläre Ca-Transient, der zur Plättchenaggregation führt, durch 3 μ-Mol der Verbindung A gehemmt wird.

In der bereits erwähnten Patentanmeldung EP-A-251 194 wird erwähnt, daS in vitro-Untersuchungen an der glatten Muskulatur (Aortenstreifen; C. van Breemen, P. Aarenson, R. Lautzenheiser, K. Meisheri, Chest 78: 157S - 165S (1980); R. Casteels und G. Droogman, J. Physio. 317: 263 - 279 (1981) ergeben haben, daß es sich bei den Verbindungen der Formel I um Calciumantagonisten mit einem neuen Wirkungsmechanismus handelt. Zur Klarstellung wird betont, daß die genannten Untersuchungen die kardiovaskuläre Wirkung der Verbindungen zeigen. Die damaligen Untersuchungsergebnisse legen jedoch in keiner Weise nahe, daß die Verbindungen der allgemeinen Formel I an inflammatorischen oder neuronalen Zellen wirken.

Der Pathophysiologie des chronischen Bronchialasthma liegen entzündliche Prozesse zugrunde, die durch die Aktivierung inflammatorischer Zellen vermittelt werden (Barnes, 1987; SEIFERT und SCHULTZ, 1991).

Die Rezeptor-regulierte Aktivierung dieser Zellen (z.B. neutrophile Granulozyten und Mastzellen bzw. deren permanente Zellinien HL 60-Zellen doer RBL-Zellen) wird unabhängig von der Art der stimulierenden Agonisten (z.B. Endothelin, PAF, Leukotriene oder chemotaktisches Peptid fMLP) durch Blocker unselektiver Kationenkanäle (UKK) inhibiert (RINK, 1990). Durch diese Kanäle gelangt extrazelluläres Calcium in die Zellen, das für die Persistenz der Rezeptor-vermittelten Zellaktivierungen erforderlich ist (PUTNEY 1990). Wird diese $Ca^{2+}$-Zufuhr unterbrochen, resultiert eine Blockade der Aktivierung inflammatorischer Zellen. Klassische Calciumantagonisten vom Dihydropyridin- bzw. vom Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorimetrisch die Kinetik der cytoplasmatischen $Ca^{2+}$-Konzentrationen in FURA 2-beladenen Zellen anhand der von GRYNKIEWICZ et al. 1985 beschriebenen Methode quantifiziert. Diese Vorgehensweise hat sich im Rahmen dieser Erfindung als zuverlässige Screeningmethode zur Auffindung von UKK-Blockern erwiesen. Verwendet werden entweder Zellsuspensionen von HL 60-Zellen oder einzelne an Glasplättchen adhärente RBL-2H3 Zellen, die stabil transfiziert sind mit menschlichen $M_1$-Rezeptoren (d.h. muskarinische Typ 1-Rezeptoren). Aufgrund der Transfizierung lassen sich die Zellen mit Carbachol stimulieren. Die konsekutiv geöffneten UKKs sind durch UKK-Blocker zu inhibieren.

Soweit die erfindungsgemäßen Verbindungen an beiden Zellsystemen untersucht wurden, zeigten sie dort übereinstimmende UKK-blockierende Effektivitäten. Weil Zellsuspensionen aus meßtechnischen Gründen nur verwendet werden können zur Austestung von Verbindungen ohne Eigenfluoreszenz, werden die fluoreszierenden Testsubstanzen an RBL-Zellen untersucht. An adhärenten Einzelzellen kann die Eigenfluoreszenz vernachlässigt werden. Die Kultivierung, Differenzierung, FURA 2-Beladung der HL 60-Zellen sowie die fluorimetrische Calciummessung in diesen Zellen erfolgte in Anlehnung an die von PITTET et al. 1990 beschriebene Methode. Für die Messung wurde ein Spektrofluorimeter LS 5 von PERKLIN ELMER verwendet.

Die Kultivierung und Anhaftung der RBL-2H3-Zellen erfolgte in der von HIDE und BEAVEN (1991) beschriebenen Methode. Die fluorimetrische Calciummessung in Cytoplasma einzelner adhärenten RBL-2H3-Zellen erfolgte in Analogie zu der von KUDO und OGURA (1986) für neuronale Zellen beschriebenen Methode. Verwendet wurde ein AXIOVERT 35 FLUORESZENZMIKROSKOP von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{2+}$-Konzentration wird als Konzentrations-Zeit-Kurve nach der Rezeptorvermittelten Zellaktivierung fortlaufend aufgezeichnet. Die Fläche unter diese Kurve (area under the curve = AUC) wird integriert und als Maß der Zellaktivierung registriert. Die inhibitorische Wirkungsstärke der getesteten UKK-Blocker wird ermittelt anhand folgender Beziehung:

$$\% H = 100 - \frac{AUC_{inh} \times 100}{AUC}$$

% H = die prozentuale Hemmung des Calciumeinstroms durch unselektive Kationenkanäle der durch 30 µM Carbachol bzw. 10 nM fMLP an RBL- bzw. an HL 60-Zellen stimuliert und durch 10 µM Testsubstanz inhibiert wird.

$AUC_{inh}$ = Fläche unter der Kurve, die in Gegenwart von Stimulator plus 10 µM inhibitorischer Testsubstanz aufgezeichnet wurde. AUC = Fläche unter der Kurve, die nach Zusatz des stimulierenden Agonisten (30 µM Carbachol an RBL-Zellen bzw. 10 nM fMLP an HL 60-Zellen) registriert wurde.

Verbindungen, die den $Ca^{2+}$-Einstrom unter den genannten Versuchsbedingungen zu mehr als 50 % inhibieren, werden zur Ermittlung einer halbmaximalen Wirkstoffkonzentration weiter untersucht.

Im folgenden werden die % H-Werte der in Eindosentesten untersuchten Prüfsubstanzen aufgelistet.

Die genannten Werte sind ein Maß für die Wirkungsstärke der UKK-Blockade.

LITERATUR zu den obigen Erläuterungen:

BARNES P.J., I.W. RODGER und N.C. THOMSON
Pathogenesis of asthma, in "ASTHMA, basic mechanisms and clinical management"
ED by P.J. BARNES; ACADEMIC PRESS, LONDON, 1988
GRYNKIEWICZ G., M. POENIE und R.Y. TSIEN
A new generation of $Ca^{2+}$-indicators with greatly improved fluorescence properties
J. BIOL. CHEM. 260: 3440-3450, 1985
HIDE, M. und M.A. BEAVEN
Calcium influx in a rat mast cell (RBL-2H3) line
J. BIOL. CHEM. 266 15221-15229, 1991
KUDO, Y. und A. OGURA
Glutamate-induced increase in intracellular $Ca^{2+}$-concentration in isolated hippocampal neurones
BR. J. PHARMACOL. 89: 191-198, 1986
PITTET, D., F. DIVIRGILIO, T. POZZAN, A. MONOD und D.P. LEW
Correlation between plasma membran potential and second messenger generation in the promyleloic cell line HL60
J. BIOL. CHEM. 265: 14256-14263, 1990
PUTNEY, J.W., jr.
Capacitative Calcium entry revised
CELL CALCIUM 11: 611-624, 1990
RINK,, T.J.
Receptor-mediated calcium entry
FEBS LETT. 268: 381-385, 1990
SEIFERT, R. und G. SCHULTZ
The superoxide forming NADPH oxidase of phagocytes: An enzym system regulated by multiple mechanism
REV. PHYSIOL. BIOCHEM. PHARMACOL., Vol. 117, SPRINGER VERL., 1991
WELLS, E., C.G. JACKSON, S.T. HARPER, J. MANN and R.P. EAOY
Characterization of primate broncoalveolar mast cells II, inhibition of histamin, $LTC_4$ and $PG_2$ release from primate bronchoalveolar mast cells and a comparison with rat peritoneal mast cells
J. IMMUNOL. 137: 3941-3945, 1986.

Die in der folgenden Tabelle angegebenen Verbindungen sind in den Tabellen 1 bis 12 enthalten.

| Verbindung Nr. | %H (RBL-Zellen) |
|---|---|
| 2 | 48.70 |
| 6 | 70.78 |
| 7 | 73.42 |
| 10 | 85.42 |
| 16 | 84.05 |
| 22 | 82.97 |
| 23 | 75.62 |
| 24 | 70.84 |
| 25 | 78.04 |
| 26 | 68.64 |
| 27 | 74.52 |
| 30 | 33.94 |
| 32 | 73.3 |
| 37 | 87.44 |
| 38a=A | 61.16 |
| 42 | 96.74 |
| 45 | 76.61 |
| 46 | 84.50 |
| 47 | 74.13 |
| 57 | 48.85 |
| 83 | 39.60 |
| 87 | 75.04 |
| 89 | 92.69 |
| 91 | 67.1 |
| 92 | 62.6 |

Aus diesen Ergebnissen kann geschlossen werden, daß für die Behandlung chronisch inflammatorischer Prozesse, insbesondere des chronischen Bronchialasthmas Verbindungen der allgemeinen Formel I besonders geeignet sind, worin

A

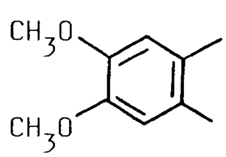 , 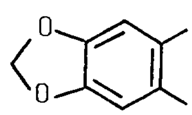 oder 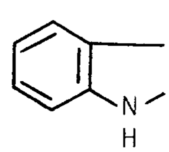

bedeutet,
und/oder
X die Gruppe $-NHR_2$ ist, worin
$R^2$ Alkyl mit 3, 4 oder 5 Kohlenstoffatomen, oder

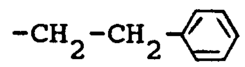

ist(worin die Phenylgruppe durch eine, zwei oder 3 Methoxygruppen oder -O-$CH_2$-O-substituiert sein kann) oder

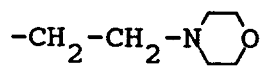

oder X substituiertes

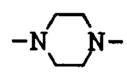

ist,
insbesondere worin X

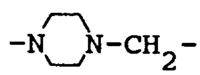

ist.

Die Verbindungen können oral, parenteral oder topisch verabreicht werden. Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen können erfindungsgemäß enteral, parenteral oder topisch verabreicht werden, vorteilhaft in einer Menge von 0,05 bis 500 mg pro Dosis für erwachsene Menschen. Vorzugsweise werden bei oraler Verabreichung 0,1 bis 500 mg pro Dosis, und bei i.v. Verabreichung 0,05 bis 150 mg pro Dosis eingesetzt.

Formulierungsbeispiele

| Beispiel 1: Tabletten | |
|---|---|
| Wirkstoff gemäß Erfindung | 40,0 mg |
| Milchzucker | 100,0 mg |
| Maisstärke | 50,0 mg |
| kolloidale Kieselsäure | 2,0 mg |
| Magnesiumstearat | 3,0 mg |
| insgesamt | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

| Beispiel 2: Dragées | |
|---|---|
| Wirkstoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Megnesiumstearat | 3,0 mg |
| insgesamt | 195,0 mg |

Herstellung:

Der Wirkstoff und die Hilfsstoffe werden, wie in Beispiel 1 beschrieben, zu Tablettenkernen verpreßt, die mit Zukker, Talcum und Gummi arabicum in üblicher Weise dragiert werden.

| Beispiel 3: Suppositorien | |
|---|---|
| Wirkstoff gemäß Erfindung | 50,0 mg |
| Milchzucker | 250,0 mg |
| Suppositorienmasse q.s. ad | 1,7 g |

Herstellung:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

| Beispiel 4: Ampullen | |
|---|---|
| Wirkstoff gemäß Erfindung | 20,0 mg |
| Natriumchlorid | 5,0 mg |
| Bi-destilliertes Wasser q.s. ad | 2,0 ml |

Herstellung:

Der Wirkstoff und das Natriumchlorid werden in bi-destilliertem Wasser gelöst und die Lösung in Ampullen steril abgefüllt.

| Beispiel 5: Ampullen | |
|---|---|
| Wirkstoff gemäß Erfindung | 10,0 mg |
| Natriumchlorid | 7,0 mg |
| Bi-destilliertes Wasser q.s. ad | 1,0 mg |

| Beispiel 6: Tropfen | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q.s. ad | 100,00 ml |

Herstellung:

Der Wirkstoff und die Konservierungsmittel werden in entmineralsiertem Wasser gelöst, die Lösung filtriert und in Flaschen zu je 100 ml abgefüllt.

Wie bereits oben erwähnt worden ist, sind die Verbindungen der allgemeinen Formel Ie neu. Sie können analog zu den in EP-A-00 37 934 und EP-A-251 194 beschriebenen Verfahren hergestellt werden:

a) durch Cyclisierung eines Phenylmalonsäurediamids der Formel III

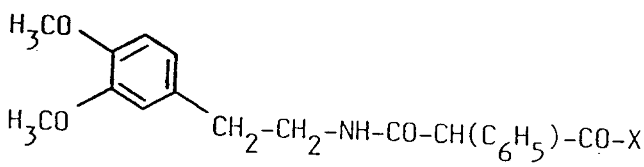

III

in Gegenwart eines Kondensationsmittels
oder
b) ausgehend von Carbonsäuren oder Carbonsäurehalogeniden der Formel IV, in der Y für OH oder Halogen steht, durch Umsetzung mit dem Amin der allgemeinen Formel VI

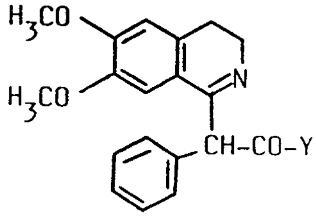

\+ XH

IV VI

in Gegenwart eines geeigneten Kondensationsmittels.

In den obigen Formeln III und IV ist die Gruppe X wie für Formel Ie definiert.

Als Kondensationsmittel für das erfindungsgemäße Verfahren a) eignen sich zahlreiche Lewissäuren, wie z.B. Phosphoroxichlorid, Bortrifluorid, Zinntetrachlorid oder Titantetrachlorid, aber auch starke Mineralsäuren wie Schwefelsäure, Fluorsulfonsäuren, Fluorwasserstoffsäure oder Polyphosphorsäure. Sie werden gewöhnlich im Überschuß eingesetzt. Bevorzugt wird Phosphoroxichlorid.

Die Cyclisierungsreaktion kann in Gegenwart oder in Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen. Beispiele sind Benzol, Alkylbenzole, Chlorbenzole, Dekalin, Chloroform, Methylenchlorid, Acetonitril und dergl. Eine Variante des Verfahrens besteht darin, das Kondensationsmittel, beispielsweise Phosphoroxichlorid, selbst als Lösungsmittel zu verwenden.

Bezüglich der Reaktionstemperatur bestehen keine speziellen Bedingungen. Die erfindungsgemäße Umsetzung kann innerhalb eines großen Temperaturbereiches, vorzugsweise unter Erwärmen oder Erhitzen bis etwa zum Siedepunkt des Lösungsmittels durchgeführt werden.

Die Amidierungsreaktion b) kann prinzipiell unter den gleichen Bedingungen wie Reaktion a) durchgeführt werden. Als Kondensationsmittel sind zusätzlich Carbodiimide - wie Cyclohexylcarbodiimid - oder Carbonyldiimidazol zu nennen.

<u>Beispiel</u>

3,4-Dihydro-1-benzyl-6,7-dimethoxy-α-[di-2-(2,3,4-trimethoxyphenyl)ethyl]aminocarbonyl-isochinolinhydrochlorid

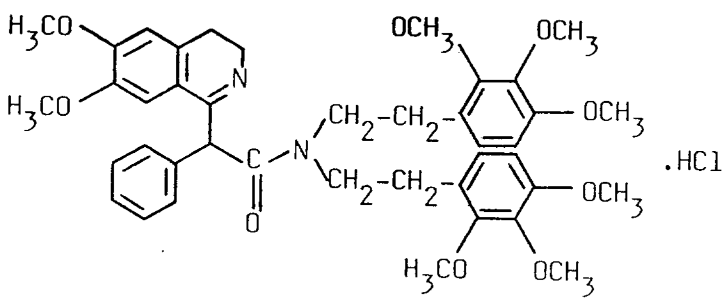

a) 2-(3,4-Dimethoxyphenyl)ethylaminocarbonyl-phenylessigsäure-N,N-di-[2-(2,3,4-trimethoxyphenyl)ethyl]amid

In eine Lösung von 18,0 g (52,4 mmol) Phenylmalonsäuremonoethylester-2-(3,4-dimethoxyphenyl)ethylamid in 150 ml wasserfreiem Dimethylformamid werden bei Raumtemperatur portionsweise 9,0 g (55,5 mmol) N,N'-Carbonyldiimidazol eingerührt. Nach 30 Min. werden 18,0 g (44,3 mmol) Di-[2-(2,3,4-Trimethoxyphenyl)ethyl]amin zugegeben und 30 Min gerührt. Danach wird das Lösungsmittel im Vakuum abdestilliert, der Rückstand in 1,5 l $CH_2Cl_2$ aufgenommen und nacheinander jeweils 2 mal mit 250 ml Wasser und 200 ml 1N HCl geschüttelt. Die organische Phase wird nach dem Trocknen über $Na_2SO_4$ eingeengt, und der Rückstand nach der Reinigung über eine Kieselgelsäule (Eluens: $CH_2Cl_2$/MeOH 100:2) aus Essigester/Ether kristallisiert.
Ausbeute: 35,5 g

b) 35,0 g (47,5 mmol) Amid (aus Stufe a) und 15 ml (164 mmol) Phosphoroxychlorid werden in 150 ml wasserfreiem Acetonitril 30 Minuten zum Sieden erhitzt. Nach beendeter Umsetzung (Dünnschichtchromatographie-Kontrolle) werden das Lösungsmittel und nicht verbrauchtes Phosphoroxychlorid im Vakuum abdestilliert. Der Rückstand wird mit Eiswasser versetzt, mit Sodalösung alkalisch gestellt und mit ca. 1 l $CH_2Cl_2$ portionsweise extrahiert. Die organische Phase wird mit Wasser gewaschen, über $Na_2SO_4$ getrocknet und eingeengt. Der Rückstand wird zweimal über eine Kieselgelsäule (1. Eluens: $CH_2Cl_2$:MeOH 100:2 → 100:4 aufsteigend; 2. Eluens: $CH_2Cl_2$/Essigester 1:1) gereinigt.

Vom gereinigten Produkt (6,5 g) wird durch Lösen in ca. 50 ml Ethanol und Zugabe alkoholischer Salzsäure das Hydrochlorid gebildet. Nach dem Einengen und Trocknen im Hochvakuum bei 50°C verbleiben 11,5 g des gewünschten Produkts. (Fp. 56-64°C, amorph)

Analog können zum Beispiel auch die Verbindungen der Tabelle 12 hergestellt werden.

## Patentansprüche

1. Verwendung von carbocyclisch und heterocyclisch anellierten Dihydropyridinen der Formel

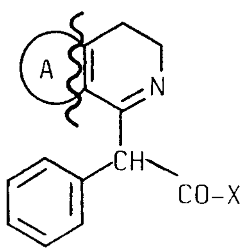

sowie deren tautomere Formen der Formel

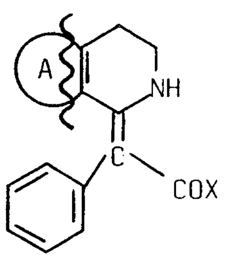

oder

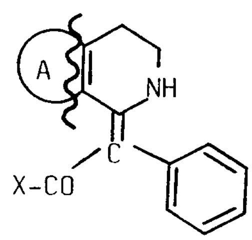

II' II''

II

in der X für $OR_1$; $NHR_2$; $NR_3R_4$
und

$R_1$ für Wasserstoff, $C_1$-$C_4$-Alkyl; $C_1$-$C_4$-Alkoxyalkyl;
$R_2$ für Wasserstoff, $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c) die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann).
b) Phenyl; gewünschtenfalls ein- oder mehrfach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe $-O-(CH_2)_n-O$ (mit n = 1 oder 2)
c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können) $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

oder $R_2$ Phenyl ist, das wie oben unter (b) angegeben substituiert sein kann;

$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1) substituiert sein kann;

und

$R_5$ für ein Phenylradikal, welches gewünschtenfalls wie vorstehend unter b) substituiert ist, steht;
A steht für die anellierten Ringsysteme

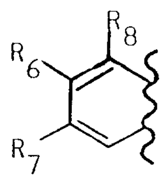

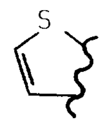

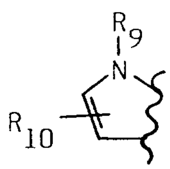

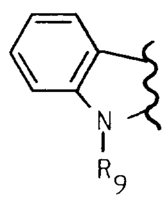

in denen
$R_8$ für Wasserstoff; $C_1$-$C_4$-Alkyl; oder $C_1$-$C_4$-Alkoxy
$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für -O-$(CH_2)_n$-O- (mit n = 1 oder 2)
$R_9$ für Wasserstoff; $C_1$-$C_4$-Alkyl
$R_{10}$ für Wasserstoff; oder 2-Phenyl-2-ethoxycarbonylacetyl;

steht sowie deren Salze mit physiologisch verträglichen Säuren, Basen oder Komplexbildnern für die Herstellung von Mitteln für die Behandlung chronisch inflammatorischer Prozesse.

2. Verwendung von carbocylisch und heterocyclisch anellierten Dihydropyridinen der Formel I bzw. II nach Anspruch 1,
in der X für $OR_1$ $NHR_2$; $NR_3R_4$

$R_1$ für Methyl oder Ethyl
$R_2$ für Wasserstoff; geradkettiges oder verzweigtes unsubstituiertes $C_1$-$C_5$-Alkyl; Allyl; Propargyl; $C_3$-$C_6$-

Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

d) Cyano, Hydroxy, Methoxy, Dimethylamino
e) Phenyl, 3,4-Methylendioxyphenyl, durch eine, zwei oder drei Methoxygruppen substituiertes Phenyl, 3-Hydroxy-4-methoxyphenyl,
f) Morpholino, Pyridin-2-yl, Indol-3-yl, Furan-2-yl, Thiophen-2-yl, Pyridin-3-yl, Pyridin-4-yl

$R_3$ und $R_4$ unabhängig voneinander für Methyl; Ethyl; 3-Cyanopropyl; Benzyl; oder 3,4,5-Trimethoxyphenethyl stehen oder

$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und

A für die anellierten Ringsysteme

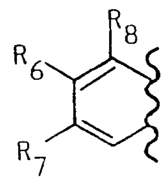

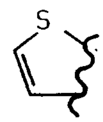

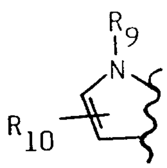

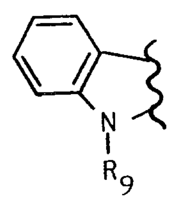

in denen

$R_8$ für Wasserstoff; oder Methoxy
$R_6$ für Methoxy; Hydroxy; Amino; oder Methansulfonylamino
$R_7$ für Wasserstoff; Methoxy; oder Hydroxy
$R_9$ für Methyl
$R_{10}$ für 2-Phenyl-2-ethoxycarbonylacetyl

steht.

**3.** Verwendung einer Verbindung der Formel I bzw. II nach Anspruch 1 oder 2, worin A der Rest

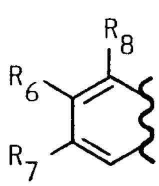

oder

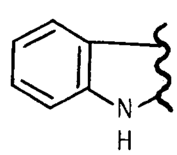

ist.

**4.** Verwendung einer Verbindung der Formel I bzw. II nach einem der Ansprüche 1 bis 3, worin $R_8$ Wasserstoff ist und $R_6$ und $R_7$ unabhängig voneinander Hydroxy oder Methoxy oder gemeinsam -O-$CH_2$-O- sind, vorzugsweise $R_6$ und $R_7$ Methoxy sind.

**5.** Verwendung einer Verbindung nach einem der Ansprüche 1 bis 4, worin X $-NHR_2$ oder $-NR_3R_4$ ist.

**6.** Verwendung einer Verbindung nach Anspruch 5, worin $X-NHR_2$ ist, worin $R_2$ $C_1$-$C_6$-Alkyl ist.

**7.** Verwendung einer Verbindung nach Anspruch 6, worin $R_2$ $C_1$-$C_4$-Alkyl ist, das unsubstituiert ist oder durch Hydroxy, Phenyl (das durch Hydroxy, Methoxy oder $-O-CH_2-O-$ substituiert sein kann) oder Morpholino substituiert ist.

**8.** Verwendung einer Verbindung nach Anspruch 1, deren Formel

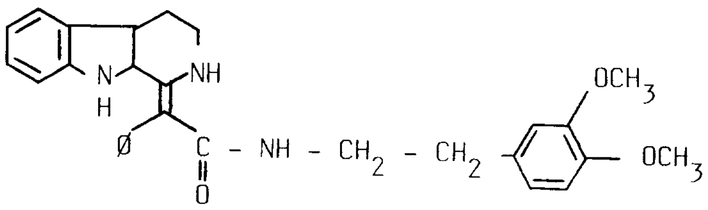

ist, oder deren pharmazeutisch annehmbares Salz.

**9.** Verwendung einer Verbindung nach Anspruch 1, deren Formel

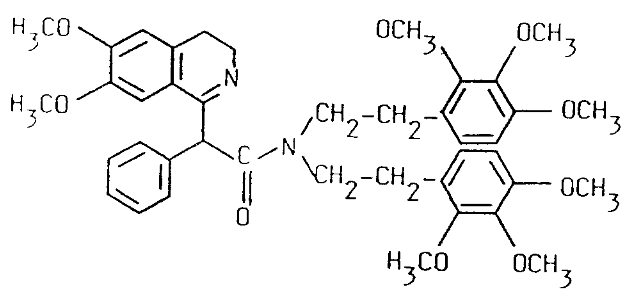

ist, oder deren pharmazeutisch annehmbares Salz.

Europäisches Patentamt

# EUROPÄISCHER RECHERCHENBERICHT

Nummer der Anmeldung
EP 96 11 9881

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP 0 288 048 A (BOEHRINGER INGELHEIM) 26.Oktober 1988<br>--- | | A61K31/435<br>A61K31/47 |
| D,A | EP 0 251 194 A (BOEHRINGER INGELHEIM) 7.Januar 1988<br>----- | | |
| | | | RECHERCHIERTE SACHGEBIETE (Int.Cl.6) |
| | | | A61K |

Der vorliegende Recherchenbericht wurde für alle Patentansprüche erstellt

| Recherchenort | Abschlußdatum der Recherche | Prüfer |
|---|---|---|
| DEN HAAG | 9.April 1997 | Klaver, T |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)